(19) Europäisches Patentamt
European Patent Office
Office européen des brevets

(11) **EP 3 171 751 B1**

(12) **EUROPÄISCHE PATENTSCHRIFT**

(45) Veröffentlichungstag und Bekanntmachung des Hinweises auf die Patenterteilung:
**06.03.2019 Patentblatt 2019/10**

(21) Anmeldenummer: **15741533.2**

(22) Anmeldetag: **21.07.2015**

(51) Int Cl.:
*A61B 1/00* (2006.01)   *A61B 1/015* (2006.01)
*A61M 5/142* (2006.01)   *A61M 5/168* (2006.01)
*A61M 5/172* (2006.01)   *A61B 5/00* (2006.01)
*A61G 13/06* (2006.01)   *A61G 13/00* (2006.01)
*A61M 3/02* (2006.01)   *G05D 16/20* (2006.01)
*A61M 1/00* (2006.01)

(86) Internationale Anmeldenummer:
**PCT/EP2015/066615**

(87) Internationale Veröffentlichungsnummer:
**WO 2016/012432 (28.01.2016 Gazette 2016/04)**

(54) **PUMPENVORRICHTUNG**

PUMP DEVICE

DISPOSITIF À POMPE

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **23.07.2014 DE 102014214359**

(43) Veröffentlichungstag der Anmeldung:
**31.05.2017 Patentblatt 2017/22**

(73) Patentinhaber: **OLYMPUS Winter & Ibe GmbH 22045 Hamburg (DE)**

(72) Erfinder: **WOLTER, Michael 22307 Hamburg (DE)**

(74) Vertreter: **Glawe, Delfs, Moll Partnerschaft mbB von Patent- und Rechtsanwälten Postfach 13 03 91 20103 Hamburg (DE)**

(56) Entgegenhaltungen:
**CN-U- 201 921 065       CN-U- 203 100 726
DE-A1- 3 540 326       DE-A1-102007 062 200
US-A- 4 795 424       US-A- 5 586 973
US-A1- 2005 077 852       US-A1- 2012 042 451
US-B1- 6 522 908**

**Beschreibung**

Die Erfindung betrifft Pumpenvorrichtungen zur Verwendung in Operationssälen, insbesondere zur Zuführung von Spülflüssigkeit bei minimal-invasiver Chirurgie, sowie Anordnungen umfassend entsprechende Pumpenvorrichtungen.

Im Bereich der Medizintechnik für Operationssäle finden sich vielfach Pumpen als Teil von medizinischen Apparaten oder als gesonderte, mit medizinischen Apparaten verbundene Einheiten. Im Bereich der minimal-invasiven Chirurgie ist es bekannt, Spülflüssigkeit in das Operationsgebiet bzw. einen das Operationsgebiet umfassenden Körperhohlraum mithilfe einer Pumpe einzubringen. Der Körperhohlraum wird dabei durch die Spülflüssigkeit so gefüllt und erweitert, dass das Operationsgebiet für den Operateur bspw. durch ein Endoskop gut sichtbar ist. Die Spülflüssigkeit kann dabei durch das Endoskop selbst zu- und ggf. auch wieder abgeführt werden. Es ist aber auch möglich, dass die Spülflüssigkeit durch vom Endoskop getrennte Vorrichtungen wie bspw. Katheter in das Operationsgebiet eingebracht wird.

Beim Spülen eines Operationsgebiets ist es von immenser Bedeutung, dass ein durch den Operateur vorgegebener Druck der Spülflüssigkeit im Operationsgebiet möglichst exakt eingehalten wird. Sinkt der Druck unterhalb des Vorgabewerts kann der das Operationsgebiet umfassende Körperhohlraum zusammenfallen. Wird in einem solchen Moment ein operativer Eingriff mit einem dafür geeigneten Instrument durchgeführt, besteht die Gefahr, dass das den Körperhohlraum bildende Gewebe mit dem Operationsinstrument in Kontakt kommt und durch dieses beschädigt wird. Liegt der Druck oberhalb des Vorgabewerts kann die Spülflüssigkeit in das Gewebe und/oder den Blutkreislauf des Patienten eindringen, was zu einer hypotonen Hyperhydratation mit Herz-Kreislauf-Belastung bis hin zu akuter Rechtsherzinsuffizienz führen kann. Es gilt solche gravierende Komplikationen unbedingt zu vermeiden.

Zur Vermeidung entsprechender Komplikationen ist es im Stand der Technik bekannt, den Pumpendruck der Pumpen für Spülflüssigkeit möglichst genau auf einen Vorgabewert - also konstant - zu halten sowie eine Bilanzierung von einströmender und abfließender Spülflüssigkeit vorzunehmen. Durch erstgenannte Maßnahme soll der Druck im Operationsgebiet weitestgehend konstant gehalten werden; die Bilanzierung der Spülflüssigkeit ermöglicht die Kontrolle, dass keine Spülflüssigkeit in das Gewebe und/oder den Blutkreislauf des Patienten eindringt.

Nachteilig an diesem Stand der Technik ist, dass während eines operativen Eingriffs auftretende Veränderungen an den Rahmenbedingungen, die Einfluss auf den benötigten und zulässigen Druck im Operationsgebiet haben, regelmäßig nicht berücksichtigt werden. Entsprechende Veränderungen erfordern im Stand der Technik grundsätzlich eine manuelle Veränderung des Vorgabewertes für den Pumpendruck, was während Operationen jedoch häufig nicht bedacht und zu den oben genannten Komplikationen führen kann.

Aus der Offenlegungsschrift DE 40 24 676 A1 ist eine Vorrichtung zum Spülen der Harnblase bekannt, bei der der notwendige Spüldruck allein aus einer erhöhten Anordnung des Spülmittelreservoirs erzeugt wird. Die Vorrichtung umfasst einen Druckregler, der ortsfest gegenüber dem Operationsgebiet angeordnet werden soll (bspw. durch Auflegen des Druckreglers auf den Bauch des Patienten), um so jegliche Druckänderung, die sich durch Veränderung des Höhenunterschiedes zwischen Druckregler und Harnblase ergeben könnte, zu vermeiden. Indem der Druckregler auch bei verschiedenen Patienten in einer gegenüber der Harnblase vergleichbaren Position angeordnet werden kann, kann mit patientenunabhängigen Erfahrungswerten bei der Einstellung des Druckreglers gearbeitet werden. Nachteilig an diesem Stand der Technik ist, dass der Druckregler unmittelbar in Kontakt mit der Spülflüssigkeit kommt und daher regelmäßig sterilisiert werden muss. Weitere Vorrichtungen zur Zufuhr von Spülflüssigkeit sind aus der US 5,586,973 oder der US 4,795,424 bekannt.

Der Erfindung liegt die Aufgabe zu Grunde, eine Pumpenvorrichtung zur Verwendung in Operationssälen, insbesondere zur Zuführung von Spülflüssigkeit bei minimal-invasiver Chirurgie, sowie Anordnungen umfassend entsprechende Pumpenvorrichtung zu schaffen, bei der die Nachteile aus dem Stand der Technik nicht mehr oder nur noch in vermindertem Umfang auftreten.

Gelöst wird diese Aufgabe durch eine Vorrichtung gemäß dem Hauptanspruch sowie eine Anordnung gemäß dem neben geordneten Anspruch 11. Vorteilhafte Weiterbildungen sind Gegenstand der abhängigen Ansprüche.

Demnach betrifft die Erfindung eine Pumpenvorrichtung zur Verwendung in Operationssälen umfassend eine Pumpeneinheit mit einer Pumpe zum Pumpen einer Spülflüssigkeit durch einen Pumpenschlauch, und einer Steuerungseinheit zur Steuerung des Pumpendrucks der Pumpeneinheit, wobei die Pumpenvorrichtung ein Erfassungsmodul zur Feststellung einer Höhenverstellung des Operationstisches des Operationssaales aufweist.

Weiterhin betrifft die Erfindung eine Anordnung aus einer Pumpenvorrichtung und einem Operationstisch, wobei die Pumpenvorrichtung erfindungsgemäß ausgestaltet ist.

Die Erfindung hat erkannt, dass sich bspw. der Druck der Spülflüssigkeit in einem Operationsgebiet bei der minimal-invasiven Chirurgie nach der initialen Vorgabe eines Pumpendrucks (bspw. durch den Operateur) insbesondere durch Höhenverstellung des Operationstisches, auf dem der zu operierende Patient liegt, ändert. Die Druckänderungen resultieren dabei aus Änderungen des statischen Drucks aufgrund der Änderung des Höhenunterschieds zwischen Pumpe und Operationsgebiet. Diese Druckänderungen können zu kritischen Drücken im Operationsgebiet führen, bei denen das, das Operationsgebiet umgebende Gewebe zusammenfällt oder Spülflüssigkeit in das Gewebe und/oder den Blutkreislauf des Patienten übertritt.

Um entsprechende Komplikationen zu vermeiden, es dem Operateur aber gleichzeitig zu ermöglichen, den Operationstisch auch während der Operation beliebig in seiner Höhe zu verstellen, um möglichst ergonomisch arbeiten zu können, ist erfindungsgemäß vorgesehen, dass die Pumpenvorrichtung ein Erfassungsmodul zur Feststellung einer Höhenverstellung des Operationstisches aufweist. Indem die Pumpenvorrichtung ein entsprechendes Erfassungsmodul aufweist, kann - wie nachfolgend erläutert - auf eine Höhenverstellung des Operationstisches reagiert werden. Ein gegenüber einfachen Pumpvorrichtungen zusätzliches, mit dem Spülmittel in Kontakt tretendes und daher regelmäßig zu desinfizierendes Element ist bei der Erfindung nicht vorgesehen.

Um eine Höhenverstellung des Operationstisches feststellen zu können, kann vorgesehen sein, dass das Erfassungsmodul zum Empfang oder zum Abfragen von Ausgabeinformationen der Steuerungselektronik des Operationstisches ausgebildet ist, um auf Basis der Ausgabeinformationen eine entsprechende Höhenverstellung festzustellen.

Moderne Operationstische weisen häufig Elektromotoren zur Höhenverstellung und ggf. weiterer Einstellungsmöglichkeiten sowie eine dazugehörige Steuerungselektronik auf, die auch zur Einbindung in ein, diverse Einrichtungen eines Operationssaals verbindendes Datennetzwerk, ausgebildet sein kann. Über ein solches Datennetzwerk können die einzelnen Einrichtungen des Operationssaals miteinander kommunizieren und Daten austauschen. Dabei kann die Steuerungselektronik des Operationstisches regelmäßig Informationen über die eingestellte Höhe des Operationstisches liefern.

Erfindungsgemäß kann vorgesehen sein, dass das Erfassungsmodul der Pumpenvorrichtung mit der Steuerungselektronik des Operationstisches über ein Datennetzwerk oder alternativ über eine direkte drahtgebundene oder drahtlose Verbindung kommunizieren kann. Die dabei von der Steuerungselektronik des Operationstisches an das Erfassungsmodul der Pumpenvorrichtung übermittelten Ausgabeinformationen können bspw. Informationen über die momentane absolute Höhe des Operationstisches, Informationen über ein momentanes Verfahren des Operationstisches in dessen Höhe, o.ä. enthalten. Im Rahmen der vorliegenden Erfindung müssen die von der Steuerungselektronik des Operationstisches zur Verfügung gestellten Ausgabeinformationen jedenfalls geeignet sein, eine Höhenverstellung des Operationstisches allein aufgrund der Ausgabeinformationen eindeutig feststellen zu können. Dies kann dabei auch durch die Feststellung einer zeitlichen Veränderung von Parametern, bspw. der Höhe des Operationstisches, erreicht werden.

Das Erfassungsmodul kann zum Empfang entsprechender Ausgabeinformationen ausgebildet sein. In diesem Fall kann die Steuerungselektronik des Operationstisches regelmäßig oder bei Bedarf - d.h. insbesondere bei einer tatsächlich Verstellung der Höhe des Operationstisches - Informationen über die Höhe des Operationstisches und/oder dessen Verstellung an das Erfassungsmodul senden, die dann dort weiter verarbeitet werden können. Alternativ oder zusätzlich ist es möglich, dass das Erfassungsmodul in regemäßigen Abständen die entsprechenden Informationen bei der Steuerungselektronik des Operationstisches abfragt.

Um eine Höhenverstellung des Operationstisches feststellen zu können, kann die Pumpenvorrichtung alternativ oder zusätzlich eine Telemetrieeinheit umfassen, die zur Detektion der Höhe oder einer Höhenverstellung des Operationstisches ausgebildet ist, wobei das Erfassungsmodul zum Empfang oder zum Abfragen von Ausgabeinformationen der Telemetrieeinheit ausgebildet ist, um auf Basis der Ausgabeinformationen eine entsprechende Höhenverstellung festzustellen.

Eine Telemetrieeinheit zur Detektion der Höhe oder einer Höhenverstellung des Operationstisches kann eine Entfernungsmessungseinheit und/oder einen Beschleunigungssensor umfassen.

Im Falle einer Entfernungsmessung kann ein Telemetriesender am höhenverstellbaren Teil des Operationstisches angeordnet sein und eine Entfernungsmessungseinheit umfassen, die den Abstand zum Boden und/oder zur Decke des Operationssaals oder zu wenigstens einem, gegenüber dem nicht in der Höhe verfahrbaren Teil des Operationstisches ortsfesten Referenzmodul bestimmen kann. Der Abstand zum Boden bzw. der Decke oder zu dem wenigstens einen Referenzmodul kann bspw. über Laufzeitmessung elektromagnetischer, insbesondere optischer, oder akustischer Wellen erfolgen, wobei die Frequenz der Wellen vorzugsweise außerhalb des sichtbaren bzw. hörbaren Spektrums liegt. Im Falle wenigstens eines Referenzmoduls kann - sofern das wenigstens eine Referenzmodul ein geeignetes messbares Feld erzeugt - die Abstandsmessung auch über eine Feldstärkenmessung erfolgen.

Durch Bestimmung des jeweiligen Abstandes zum Boden und zur Decke bzw. zu wenigstens zwei getrennt voneinander angeordneten Referenzmodulen können die jeweiligen Messungen auch gegengeprüft und so die Gefahr von Messfehlern reduziert werden. Eine Prüfung der Messergebnisse kann bspw. dadurch erfolgen, dass die beiden gemessenen Entfernungen zu Boden und Decke bzw. zu zwei Referenzmodulen in der Summe dem Abstand zwischen Boden und Decke bzw. den beiden Referenzmodulen entsprechen müssen. Ist dies nicht der Fall, ist wenigstens eine der beiden Messungen fehlerhaft und es kann ggf. eine Warnung ausgegeben werden. Eine entsprechende Prüfung und/oder Ausgabe einer Warnung kann direkt im Telemetriesender oder nachgeschaltet, bspw. durch das Empfangsmodul oder ein gesondertes Prüfmodul, erfolgen.

Es ist auch möglich, dass wenigstens ein Referenzmodul am höhenverstellbaren Teil des Operationstisches angeordnet ist und der Telemetriesender ortsfest gegenüber dem nicht in der Höhe verfahrbaren Teil des Operationstisches - bspw. dem Fuß des Operationstisches - angeordnet ist.

Es ist weiterhin möglich, dass die Telemetrieeinheit einen Beschleunigungssensor umfasst. Ist die Telemetrieeinheit am höhenverstellbaren Teil des Operationstisches angeordnet, kann über den Beschleunigungssensor eine Höhenverstellung des Operationstisches detektiert werden.

Erfindungsgemäß kann vorgesehen sein, dass das Erfassungsmodul der Pumpenvorrichtung mit dem Telemetriesender über eine drahtgebundene oder drahtlose Verbindung kommunizieren kann. Die dabei von dem Telemetriesender an das Erfassungsmodul der Pumpenvorrichtung übermittelten Ausgabeinformationen können dabei den Ausgabeinformationen einer Steuerungselektronik eines Operationstisches entsprechen. Es wird daher auf die obigen Ausführungen verwiesen.

Das Erfassungsmodul kann zum Empfang entsprechender Ausgabeinformationen ausgebildet sein. In diesem Fall kann der Telemetriesender regelmäßig oder bei Bedarf - d.h. insbesondere bei einer tatsächlich Verstellung der Höhe des Operationstisches - Informationen über die Höhe des Operationstisches und/oder dessen Verstellung an das Erfassungsmodul senden, die dann dort weiter verarbeitet werden können.

Alternativ oder zusätzlich ist es möglich, dass das Erfassungsmodul in regemäßigen Abständen die entsprechenden Informationen bei dem Telemetriesender abfragt.

Die Pumpenvorrichtung kann vorzugsweise zur Ausgabe eines optischen oder akustischen Warnsignals bei Feststellung einer Höhenverstellung des Operationstisches ausgebildet sein. Dazu kann die Pumpenvorrichtung ein oder mehrere geeignete Signalgeber umfassen. Es ist aber auch möglich, dass die Pumpenvorrichtung ein Kommunikationsmodul zur Ausgabe eines optischen oder akustischen Warnsignals auf einem externen Gerät, welches einen geeigneten Signalgeber aufweist, umfasst. Bei dem externen Gerät, mit dem die Pumpenvorrichtung für die Ausgabe von Warnungen kommuniziert, kann es sich bspw. um ein bei der Operation verwendetes Gerät handeln, das sich eher im Blickfeld des Operateurs befindet als die Pumpenvorrichtung, wie bspw. ein Vitalfunktionsmonitor.

Es ist besonders bevorzugt, wenn das Erfassungsmodul nicht nur zur Feststellung einer Höhenverstellung ausgebildet ist, sondern auch die Höhendifferenz der Höhenverstellung erfassen kann. In anderen Worten soll das Erfassungsmodul nicht nur die Tatsache, dass der Operationstisch in der Höhe verstellt wird, feststellen, sondern auch die Richtung und den Betrag der Höhenverstellung erfassen können. Die Höhendifferenz kann insbesondere ausgehend von der Höhe des Operationstisches zum Zeitpunkt der initialen Einstellung des Pumpendrucks ermittelt werden. Es ist aber auch möglich, dass die Höhendifferenz ausgehend von einer dauerhaft festgelegten Bezugshöhe ermittelt wird.

Die vorbeschriebenen Varianten zur Feststellung der Höhenverstellung des Operationstisches über die Steuerungselektronik des Operationstisches und/oder eine Telemetrieeinheit können zur Erfassung der Höhendifferenz geeignet ausgebildet sein.

Es ist bevorzugt, wenn die Pumpenvorrichtung eine Anzeige für die Höhendifferenz der Höhenverstellung aufweist. Die angezeigte Höhendifferenz kann dann dem Operateur einen Hinweis geben, ob und wenn ja in welchem Umfang der Vorgabewert für den Pumpendruck verändert werden muss.

Es ist besonders bevorzugt, wenn die Steuerungseinheit zur Änderung des Pumpendrucks in Abhängigkeit der Höhendifferenz der Höhenverstellung ausgebildet ist. Ausgehend von der durch den Operateur vorgenommenen initialen Einstellung des Pumpendrucks bei einer initialen Höhe des Operationstisches kann dann die Steuerungseinheit den Pumpendruck so regulieren, dass selbst bei einer Höhenverstellung des Operationstisches im Operationsgebiet ein gleichbleibender Druck der Spülflüssigkeit gewährleistet wird. Alternativ ist es möglich, dass der Operateur den Pumpendruck bereits bezogen auf eine Bezugshöhe vorgibt und die Steuerungseinheit den Pumpendruck gemäß der Höhendifferenz anpasst.

Der Zusammenhang zwischen Höhendifferenz, der Höhenverstellung und der Änderung des Pumpendrucks ist vorzugsweise linear. Weiter Vorzugsweise entspricht dieser Zusammenhang der Gleichung:

$$\Delta p = a \times \Delta h \times 10^4 \frac{Pa}{m}$$

wobei $\Delta p$ der Änderung des Pumpendrucks und $\Delta h$ die Höhendifferenz der Höhenverstellung entspricht. Bei dem Term $10^4 \frac{Pa}{m}$ (entspricht $\frac{mbar}{cm}$) handelt es sich um ein Umrechnungsfaktor, der den statischen Druck typischer Spülflüssigkeit, insbesondere wasserbasierter Spülflüssigkeit, in Abhängigkeit von der Höhe der Flüssigkeitssäule wenigstens näherungsweise wiederspiegelt. Der Korrekturfaktor $a$ kann zwischen 0,8 bis 1,2, vorzugsweise zwischen 0,9 und 1,1 und weiter vorzugsweise 1 sein. Umrechnungsfaktor und Korrekturfaktor können auch zu einem Faktor zusammengefasst sein.

Erfindungsgemäß weist das Erfassungsmodul ein Lichtelement - vorzugsweise einen Laser - zur Projektion einer Kalibrierungsebene, einer Kalibrierungsskala oder wenigstens eines Kalibrierungspunkts auf den Operationstisch oder auf den auf dem Operationstisch liegenden Patienten auf. Die Kalibrierungsskala kann mehrere parallele Linien bspw. im Abstand von jeweils 1 cm umfassen. Indem eine entsprechende Kalibrierungsebene, eine Kalibrierungsskala oder wenigstens ein Kalibrierungspunkt projiziert wird, kann eine Höhenverstellung des Operationstisches verdeutlich werden, um dadurch den Operateur auf die Notwendigkeit der Nachjustierung des Pumpendrucks hinzuweisen. Dies ist besonders wirkungsvoll, wenn die Kalibrierungsebene, die Kalibrierungsskala oder der wenigstens eine Kalibrierungspunkt derart projiziert wird, dass in der initialen Höheneinstellung des Operationstisches die Umgebung des Operationsgebietes frei von Projektionen des Lichtelementes ist, bei einer Höhenverstellung des Operationstisches die Projektionen jedoch in diese Umgebung, die typischerweise im Blickfeld des Operateurs ist, wandern.

Zur Erläuterung der erfindungsgemäßen Anordnung wird auf die vorstehenden Ausführungen verwiesen.

Die Erfindung wird nun anhand vorteilhafter Ausführungsformen unter Bezugnahme auf die beigefügten Zeichnungen beispielhaft beschrieben. Es zeigen:

Figur 1    ein erstes Ausführungsbeispiel einer erfindungsgemäßen Anordnung umfassend ein erstes Ausführungsbeispiel einer erfindungsgemäßen Pumpenvorrichtung;

Figur 2    ein zweites Ausführungsbeispiel einer erfindungsgemäßen Anordnung umfassend ein zweites Ausführungsbeispiel einer erfindungsgemäßen Pumpenvorrichtung; und

Figur 3    eine Variante des Ausführungsbeispiels aus Figur 2 .

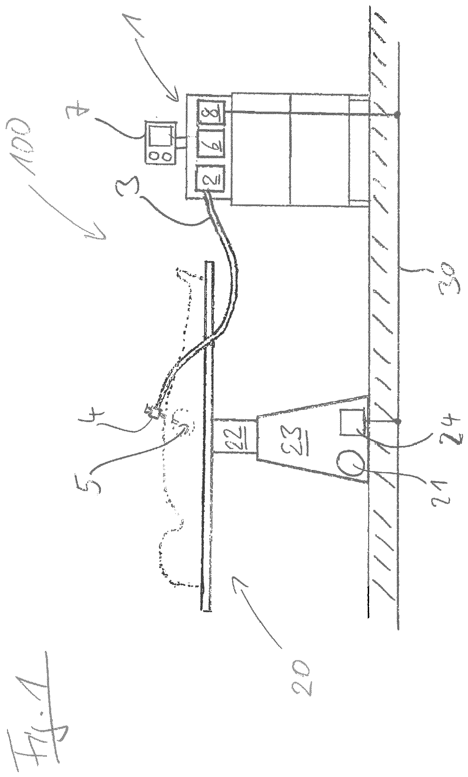

In Figur 1 ist ein erstes Ausführungsbeispiel einer erfindungsgemäßen Anordnung 100 umfassend ein erstes Ausführungsbeispiel einer erfindungsgemäßen Pumpenvorrichtung 1 sowie einem höhenverstellbaren Operationstisch 50 dargestellt.

Die Pumpenvorrichtung 1 umfasst eine Pumpeneinheit 2 zum Pumpen einer Spülflüssigkeit durch den Pumpenschlauch 3, der mit seinem einen Ende an der Pumpeneinheit 2 befestigt ist. An seinem anderen Ende ist der Pumpenschlauch 3 mit einem Endoskop 4 verbunden. Das Endoskop 4 kann für einen minimal-invasiven Eingriff in einen Körperhohlraum eines Patenten eingeführt sein, wobei der der Körperhohlraum durch die Spülflüssigkeit gefüllt und erweitert wird, sodass das dadurch freigelegte Operationsgebiet 5 für den Operateur durch das Endoskop 4 gut sichtbar ist.

Die Pumpenvorrichtung 1 weist weiterhin eine Steuerungseinheit 6 zur Steuerung des Pumpendrucks der Pumpeneinheit 2 auf. Die Steuerungseinheit 6 ist dabei dazu ausgebildet, den Pumpendruck - d.h. den von der Pumpeneinheit 2 auf der Druckseite erzeugten Druck - auf einen Vorgabewert zu regeln. Der Vorgabewert kann über eine Benutzerschnittstelle 7 eingestellt werden. Die Steuerung bzw. Regelung der Pumpeneinheit 2 kann aufgrund von eingespeicherten Betriebskurven oder anhand eines an der Druckseite der Pumpeneinheit 2 angeordneten Drucksensors (nicht dargestellt) erfolgen.

An der Pumpenvorrichtung 1 ist weiterhin ein Erfassungsmodul 7 zur Feststellung einer Höhenverstellung des Operationstisches 20 vorgesehen.

Der Operationstisch 20 ist über einen Motor 21 in der Höhe verstellbar. Dabei wird ein in der Höhe verstellbarer Teil 22 des Operationstisches 20 gegenüber einem nicht verstellbaren Teil - dem Fuß 23 - des Operationstisches 22 verfahren. Das Verfahren der beiden Teile 22, 23 zueinander wird über den Motor 21 erreicht, der von der Steuerungselektronik 24 angesteuert wird.

Die Steuerungselektronik 24 ist an ein Datennetzwerk 30 angeschlossen, über das diverse Apparate im Operationssaal (nicht dargestellt) miteinander kommunizieren und Daten austauschen können. Ebenfalls an dieses Datennetzwerk 30 angeschlossen ist die Pumpenvorrichtung 1 bzw. das Erfassungsmodul 8 der Pumpenvorrichtung 1.

Die Steuerungselektronik 24 des Operationstisches 20 ist dazu ausgebildet, im Falle einer Höhenverstellung des Operationstisches 20 ein Höhenverstellungssignal als Ausgabeinformation an das Erfassungsmodul 8 der Pumpenvorrichtung 1 zu übermitteln. Weiterhin ist Steuerungselektronik 24 dazu ausgebildet, auf Anfrage die absolute Höhe des Operationstisches 20 bzw. die relative Lage zwischen den beiden relativ zueinander beweglichen Teilen 22, 23 des Operationstisches 20 auszugeben.

Das Empfangsmodul 8 ist sowohl zum Empfang der Ausgabeinformation des Höhenverstellungssignals von der Steuerungselektronik 24 als auch zur Abfrage der Ausgabeinformationen betreffend die absolute Höhe des Operationstisches 20 von der Steuerungselektronik 24 ausgebildet.

Das Erfassungsmodul 8 der Pumpenvorrichtung 1 weist im dargestellten Ausführungsbeispiel wenigstens zwei Betriebsmodi auf, die über die Benutzerschnittstelle 7 eingestellt werden können.

In einem ersten Betriebsmodus stellt das Erfassungsmodul 8 auf Basis des von der Steuerungselektronik 24 als Ausgabeinformation übermittelten Höhenverstellungssignals eine Höhenverstellung des Operationstisches 20 fest und gibt über die Benutzerschnittstelle 7 ein Warnsignal aus. Das Warnsignal kann dabei optisch und/oder akustisch sein. Durch das Warnsignal wird der Operateur aufgefordert, den Pumpendruck der Pumpeneinheit 2 über die Steuerungseinheit 6 und die Benutzerschnittstelle 7 so anzupassen, dass im Operationsgebiet 5 ein zulässiger Druck herrscht.

In einem zweiten Betriebsmodus fragt das Erfassungsmodul 8 in regelmäßigen zeitlichen Abständen Ausgabeinformationen betreffend die absolute Höhe des Operationstisches 20 von dessen Steuerungselektronik 24 ab. Die zeitlichen Abstände der Abfragen können variabel gewählt werden. Insbesondere kann bei Empfang einer Ausgabeinformation umfassend ein Höhenverstellungssignal der zeitliche Abstand zwischen zwei Abfragen gering gewählt werden, während in der übrigen Zeit der Abstand größer gewählt ist.

Das Erfassungsmodul 8 ist dabei dazu ausgebildet, ausgehend von der Höhe des Operationstisches 20 zu dem Zeitpunkt, an dem der Pumpendruck der Pumpeneinheit 2 an der Benutzerschnittstelle 7 initial eingestellt wurde, auf Basis der abgefragten Ausgabeinformation die Höhendifferenz der Höhenverstellung - d.h. die Differenz in der Höhe des Operationstisches 20 zu einem beliebigen Zeitpunkt und dem Zeitpunkt, dem der Pumpendruck der Pumpeneinheit 2 initial eingestellt wurde - zu ermitteln. Alternativ kann die Höhendifferenz auch gegenüber einer festgelegten Bezugshöhe ermittelt werden.

Die Steuerungseinheit 6 ist dazu ausgebildet, den Pumpendruck der Pumpeneinheit 2 in Abhängigkeit von der durch das Erfassungsmodul 8 ermittelten Höhendifferenz anzupassen, sodass der Druck der Spülflüssigkeit im Operationsgebiet 5 trotz Höhenverstellung des Operationstisches 20 konstant bleibt. Dazu wird der Pumpendruck gemäß der folgenden Gleichung angepasst:

$$\Delta p = a \times \Delta h \times 10^4 \frac{Pa}{m}$$

Dabei ist $\Delta p$ die erforderliche Änderung des Pumpendrucks und $\Delta h$ die vom Erfassungsmodul 8 ermittelte Höhendifferenz. Beim Term $10^4 \frac{Pa}{m}$ (entspricht $\frac{mbar}{cm}$ ) handelt es sich um einen Umrechnungsfaktor, der Korrekturfaktor *a* ist im dargestellten Ausführungsbeispiel 1.

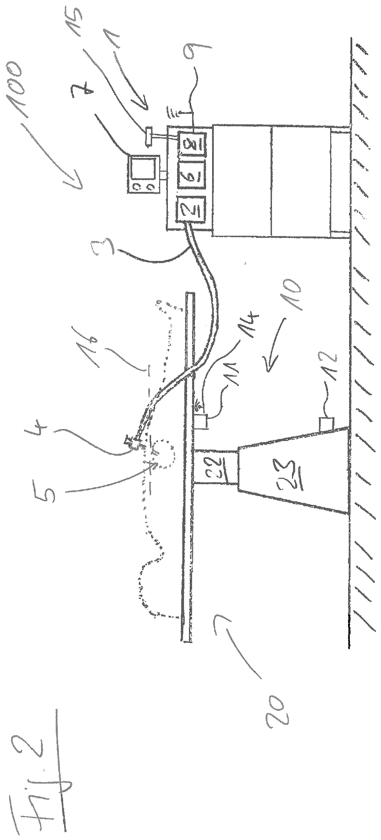

In Figur 2 ist ein zweites Ausführungsbeispiel einer erfindungsgemäßen Anordnung 100 umfassend ein zweites Ausführungsbeispiel einer erfindungsgemäßen Pumpenvorrichtung 1 sowie einem höhenverstellbaren Operationstisch 50 dargestellt.

Die Anordnung 100 und die Pumpenvorrichtung 1 aus Figur 2 entsprechen in weiten Teilen denjenigen aus Figur 1, weshalb auf die dortigen Ausführungen verwiesen wird. Nachfolgend wird lediglich auf die Unterschiede zwischen den beiden Ausführungsbeispielen gemäß Figuren 1 und 2 eingegangen.

Bei dem Ausführungsbeispiel gemäß Figur 2 ist die Erfassungseinheit 8 nicht mit einer Steuerungselektronik (24; vgl. Figur 1, in Figur 2 nicht dargestellt) des Operationstisches 20 verbunden. Vielmehr umfasst die Pumpenvorrichtung 1 noch eine Telemetrieeinheit 10 umfassend einen Telemetriesender 11 und ein Referenzmodul 12.

Der Telemetriesender 11 ist dazu ausgebildet, über eine Entfernungsmessungseinheit den Abstand zwischen ihm und dem Referenzmodul 12 zu bestimmten. Die Entfernungsmessung erfolgt dabei durch eine Feldstärkenmessung vom Referenzmodul 12 erzeugten elektromagnetischen Feldes durch die Entfernungsmessungseinheit.

Da der Telemetriesender 11 am höhenverstellbaren Teil 22 des Operationstisches 20 und das Referenzmodul 12 am Fuß 23 des Operationstisches 20 angeordnet ist, lässt sich aus dem ermittelten Abstand zwischen Telemetriesender 11 und Referenzmodul 12 die momentan eingestellte Höhe des Operationstisches 20 ermitteln. Die Anordnung von Telemetriesender 11 und Referenzmodul 12 kann dabei im Übrigen auch vertauscht sein.

Der Telemetriesender 11 weist eine Funkantenne 13 auf, die mit einer an dem Erfassungsmodul 8 angeordneten Funkantenne 9 zusammenwirkt, um Informationen zwischen Telemetriesender 11 und Erfassungsmodul 8 auszutauschen. Der Telemetriesender 11 kann dabei verschiedene Ausgabeinformationen liefern, die insbesondere mit dem Höhenverstellungssignal der Steuerungselektronik 24 aus dem ersten Ausführungsbeispiel gemäß Figur 1 als auch mit Ausgabeinformationen betreffend die absolute Höhe des Operationstisches 20 von der Steuerungselektronik 24 desselben Ausführungsbeispiels unmittelbar vergleichbar sind. Zur Generierung eines Höhenverstellungssignals kann der Telemetriesender 11 die Änderung der absoluten Höhe überwachen oder einen Beschleunigungssensor aufweisen, der jegliche Beschleunigungen in vertikaler Richtung aufnimmt.

Im zweiten Ausführungsbeispiel weist das Erfassungsmodul 8 der Pumpenvorrichtung 1 ebenfalls zwei Betriebsmodi auf, die über die Benutzerschnittstelle 7 eingestellt werden können. Die Betriebsmodi entsprechen dabei denjenigen des ersten Ausführungsbeispiels, wobei die Ausgabeinformationen lediglich von der Telemetrieeinheit 10 und nicht von der Steuerungselektronik 24 des Operationstisches 20 stammen. Es wird daher auf die obenstehenden Ausführungen verwiesen.

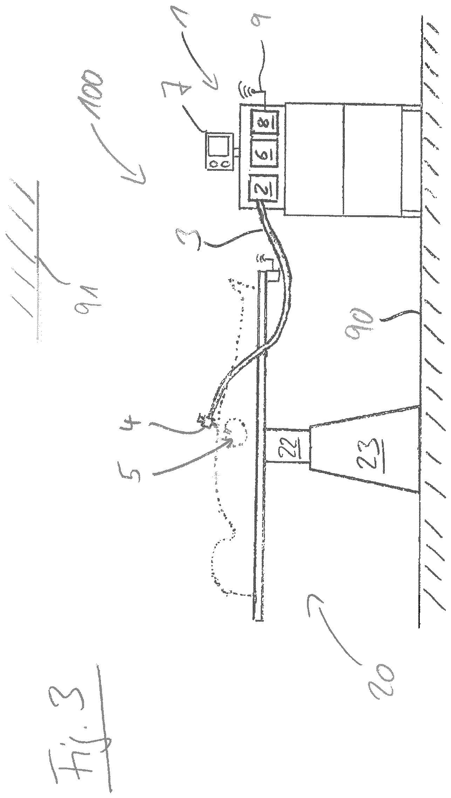

In Figur 3 ist eine Variante des Ausführungsbeispiels aus Figur 2 dargestellt.

Bei dem Ausführungsbeispiel in Figur 3 misst die Entfernungsmessungseinheit des am höhenverstellbaren Teil 22 des Operationstisches 20 angeordneten Telemetriesenders 11 den Abstand sowohl zum Boden 90 als auch zur Decke 91 des Operationssaals. Die Entfernungsmessung erfolgt dabei über eine Laufzeitmessung einer elektromagnetischen Welle außerhalb des sichtbaren Spektrums. Der Telemetriesender 11 übermittelt beide Entfernungen als Ausgabeinformation an das Erfassungsmodul 8. Das Erfassungsmodul 8 führt eine Validitätsprüfung der Entfernungen durch, indem sie die Summe der Entfernungen mit dem Abstand zwischen Boden 90 und Decke 91 vergleicht. Entspricht die genannte Summe diesem Abstand gelten die gemessenen Entfernungen als valide und können erfindungsgemäß weiterverarbeitet werden. Diesbezüglich wird auf die Ausführungen zu Figur 2 verwiesen.

Zusätzlich weist die Pumpenvorrichtung 1 in der Variante gemäß Figur 3 eine Lichtquelle 15 auf, die als Laser ausgebildet und dem Erfassungsmodul 8 zugeordnet ist. Die Lichtquelle projiziert eine Kalibrierungsebene 16 auf den auf dem Operationstisch 20 liegenden Patienten. Die Kalibrierungsebene 16 wird dabei derart projiziert, dass in der initialen Höheneinstellung des Operationstisches 20 die Umgebung des Operationsgebietes 5 - d.h. der Bereich um die Einführungsstelle des Endoskops 4 - frei von Projektionen des Lichtelementes 15 ist, bei einer Höhenverstellung des Operationstisches 20 nach unten die Projektion jedoch in diese Umgebung, die typischerweise im Blickfeld des Operateurs ist, wandert.

**Patentansprüche**

1. Pumpenvorrichtung (1) zur Verwendung in Operationssälen umfassend eine Pumpeneinheit (2) mit einer Pumpe zum Pumpen einer Spülflüssigkeit durch einen Pumpenschlauch (3) und eine Steuerungseinheit (6) zur Steuerung des Pumpendrucks der Pumpeneinheit (2), wobei die Pumpenvorrichtung (1) ein Erfassungsmodul (8) zur Feststellung einer Höhenverstellung eines Operationstisches (20) des Operationssaales aufweist, **dadurch gekennzeichnet, dass** das Erfassungsmodul ein Lichtelement (15) zur Projektion einer Kalibrierungsebene (16), einer Kalibrierungsskala oder wenigstens eines Kalibrierungspunkts auf den Operationstisch (20) oder auf den auf dem Operationstisch (20) liegenden Patienten aufweist.

2. Pumpenvorrichtung nach Anspruch 1, **dadurch gekennzeichnet, dass** das Erfassungsmodul (8) zum Empfang oder zum Abfragen von Ausgabeinformationen einer Steuerungselektronik (24) des Operationstisches (20) und zur Feststellung einer Höhenverstellung des Operationstisches (20) auf Basis der Ausgabeinformationen ausgebildet ist.

3. Pumpenvorrichtung nach Anspruch 1 oder 2, **dadurch gekennzeichnet, dass** die Pumpenvorrichtung (1) eine Telemetrieeinheit (10) mit einem Telemetriesender (11) umfasst, die zur Detektion der Höhe oder einer Höhenverstellung des Operationstisches (20) ausgebildet ist, wobei das Erfassungsmodul (8) zum Empfang oder zum Abfragen von Ausgabeinformationen des Telemetriesenders (11) der Telemetrieeinheit (10) und zur Feststellung einer Höhenverstellung des Operationstisches (20) auf Basis der Ausgabeinformationen ausgebildet ist.

4. Pumpenvorrichtung nach Anspruch 3,

**dadurch gekennzeichnet, dass** die Telemetrieeinheit (11) eine Entfernungsmessungseinheit und/oder einen Beschleunigungssensor umfasst.

5. Pumpenvorrichtung nach einem der vorhergehenden Ansprüche, **dadurch gekennzeichnet, dass** die Pumpenvorrichtung (1) zur Ausgabe eines optischen oder akustischen Warnsignals bei Feststellung einer Höhenverstellung des Operationstisches (20) ausgebildet ist.

6. Pumpenvorrichtung nach einem der vorhergehenden Ansprüche, **dadurch gekennzeichnet, dass** das Erfassungsmodul (8) zur Erfassung der Höhendifferenz der Höhenverstellung ausgebildet ist.

7. Pumpenvorrichtung nach Anspruch 6, **dadurch gekennzeichnet, dass** die Steuerungseinheit (6) zur Änderung des Pumpendrucks in Abhängigkeit der Höhendifferenz der Höhenverstellung ausgebildet ist.

8. Pumpenvorrichtung nach Anspruch 7, **dadurch gekennzeichnet, dass** der Zusammenhang zwischen Höhendifferenz der Höhenverstellung und der Änderung des Pumpendrucks linear ist, vorzugsweise gemäß der Gleichung:

$$\Delta p = a \times \Delta h \times 10^4 \frac{Pa}{m}$$

mit der Änderung des Pumpendrucks $\Delta p$, dem Korrekturfaktor $a$, der Höhendifferenz der Höhenverstellung $\Delta h$ und dem Umrechnungsfaktor $10^4 \frac{Pa}{m}$ .

9. Pumpenvorrichtung nach Anspruch 8, **dadurch gekennzeichnet, dass** der Korrekturfaktor $a$ zwischen 0,8 bis 1,2, vorzugsweise zwischen 0,9 und 1,1 und weiter vorzugsweise 1 ist.

10. Pumpenvorrichtung nach einem der vorhergehenden Ansprüche, **dadurch gekennzeichnet, dass** das Lichtelement (15) einen Laser aufweist.

11. Anordnung (100) umfassend einen höhenverstellbaren Operationstisch (20) und eine Pumpenvorrichtung (1) zur Verwendung in Operationssälen, **dadurch gekennzeichnet, dass** die Pumpenvorrichtung (1) gemäß Ansprüchen 1 bis 10 ausgebildet ist.

**12.** Anordnung nach Anspruch 11,
**dadurch gekennzeichnet, dass** der Operationstisch (20) nach einem der Ansprüche 1 bis 10 ausgebildet ist.

**Claims**

**1.** Pump device (1) for use in operating rooms comprising a pump unit (2) having a pump for pumping a rinsing liquid through a pump tube (3), and a control unit (6) for controlling the pump pressure of the pump unit (2), wherein
the pump device (1) comprises a detection module (8) for detecting a height adjustment of an operating table (20) of the operating room,
**characterized in that** the detection module comprises a light element (15) for projecting a calibration plane (16), a calibration scale or at least one calibration point onto the operating table (20) or onto the patient who is lying on the operating table (20).

**2.** Pump device according to Claim 1,
**characterized in that**
the detection module (8) is designed for receiving or for requesting output information from control electronics (24) of the operating table (20) and for detecting a height adjustment of the operating table (20) on the basis of the output information.

**3.** Pump device according to one of Claims 1 or 2,
**characterized in that**
the pump device (1) comprises a telemetry unit (10) having a telemetry transmitter (11) which is designed for detecting the height or a height adjustment of the operating table (20), wherein the detection module (8) is designed for receiving or for requesting output information from the telemetry transmitter (11) of the telemetry unit (10) and for detecting a height adjustment of the operating table (20) on the basis of the output information.

**4.** Pump device according to Claim 3,
**characterized in that**
the telemetry unit (11) comprises a detection-measuring unit and/or an acceleration sensor.

**5.** Pump device according to one of the preceding claims,
**characterized in that**
the pump device (1) is designed for outputting an optical or acoustic warning signal upon detection of a height adjustment of the operating table (20) .

**6.** Pump device according to one of the preceding claims,

**characterized in that**
the detection module (8) is designed for detecting the height difference of the height adjustment.

**7.** Pump device according to Claim 6,
**characterized in that**
the control unit (6) is designed for changing the pump pressure depending on the height difference of the height adjustment.

**8.** Pump device according to Claim 7,
**characterized in that**
the relationship between the height difference of the height adjustment and the change in the pump pressure is linear, preferably according to the equation:

$$\Delta p = a \times \Delta h \times 10^4 \frac{Pa}{m}$$

having the change in the pump pressure $\Delta p$, the correction factor a, the height difference of the height adjustment $\Delta h$ and the conversion factor $10^4 \frac{Pa}{m}$ .

**9.** Pump device according to Claim 8,
**characterized in that**
the correction factor a is between 0.8 to 1.2, preferably between 0.9 and 1.1 and, further preferably, is 1.

**10.** Pump device according to one of the preceding claims,
**characterized in that** the light element (15) comprises a laser.

**11.** Arrangement (100) comprising a height-adjustable operating table (20) and a pump device (1) for use in operating rooms,
**characterized in that**
the pump device (1) is designed according to one of Claims 1 to 10.

**12.** Arrangement according to Claim 11,
**characterized in that**
the operating table (20) is designed according to one of Claims 1 to 10.

**Revendications**

**1.** Dispositif à pompe (1) destiné à être utilisé dans des salles d'opération, comprenant un bloc pompe (2) pourvu d'une pompe destinée à pomper un liquide d'irrigation à travers une tubulure de pompe (3), et un bloc de commande (6) destiné à la commande de la pression de pompage du bloc pompe (2), dans lequel le dispositif à pompe (1) comporte un module de détection (8) destiné à détecter un réglage en hauteur d'une table d'opération (20) de la salle d'opération, **caractérisé en ce que** le module de détection comporte un élément lumineux (15) destiné à projeter sur la table d'opération (20) ou le patient étendu sur la table d'opération (20), un plan d'étalonnage (16), une échelle d'étalonnage ou au moins un point d'étalonnage.

2. Dispositif à pompe selon la revendication 1, **caractérisé en ce que**
le module de détection (8) est conçu pour recevoir ou demander des informations de sortie en provenance d'une électronique de commande (24) de la table d'opération (20) et pour déterminer un réglage en hauteur de la table d'opération (20) sur la base des informations de sortie.

3. Dispositif à pompe selon la revendication 1 ou 2, **caractérisé en ce que**
le dispositif à pompe (1) comprend un bloc de télémétrie (10) pourvu d'un émetteur de télémétrie (11) qui est conçu pour détecter la hauteur ou un réglage en hauteur de la table d'opération (20), dans lequel le module de détection (8) est conçu pour recevoir ou demander des informations de sortie de l'émetteur de télémétrie (11) en provenance du bloc de télémétrie (10) et déterminer un réglage en hauteur de la table d'opération (20) sur la base des informations de sortie.

4. Dispositif à pompe selon la revendication 3, **caractérisé en ce que**
le bloc de télémétrie (11) comprend un bloc de mesure de distance et/ou un capteur d'accélération.

5. Dispositif à pompe selon l'une des revendications précédentes, **caractérisé en ce que**
le dispositif à pompe (1) est conçu pour délivrer un signal d'avertissement optique ou acoustique lorsqu'il est déterminé qu'un réglage en hauteur de la table d'opération (20) est effectué.

6. Dispositif à pompe selon l'une des revendications précédentes, **caractérisé en ce que**
le module de détection (8) est conçu pour détecter la différence de hauteur du réglage en hauteur.

7. Dispositif à pompe selon la revendication 6, **caractérisé en ce que**
le bloc de commande (6) est conçu pour modifier la pression de pompe en fonction de la différence de hauteur du réglage en hauteur.

8. Dispositif à pompe selon la revendication 7, **caractérisé en ce que**
la relation entre la différence de hauteur du réglage en hauteur et la variation de pression de pompe est linéaire, de préférence selon l'équation :

$$\Delta p = a \times \Delta h \times 10^4 \frac{Pa}{m}$$

en fonction de la variation de la pression de pompe $\Delta p$, du facteur de correction $a$, de la différence de hauteur du réglage en hauteur $\Delta h$ et du facteur de conversion $10^4 \frac{Pa}{m}$.

9. Dispositif à pompe selon la revendication 8, **caractérisé en ce que**
le facteur de correction $a$ est compris entre 0,8 et 1,2, de préférence entre 0,9 et 1,1 et plus préférablement est égal à 1.

10. Dispositif à pompe selon l'une des revendications précédentes,
**caractérisé en ce que** l'élément lumineux (15) comprend un laser.

11. Ensemble (100) comprenant une table d'opération réglable en hauteur (20) et un dispositif à pompe (1) destiné à être utilisé dans des salles d'opération, **caractérisé en ce que**
le dispositif à pompe (1) est conçu selon l'une des revendications 1 à 10.

12. Ensemble selon la revendication 11, **caractérisé en ce que**
la table d'opération (20) est conçue selon l'une des revendications 1 à 10.

Fig. 1

Fig. 2

Fig. 3

**IN DER BESCHREIBUNG AUFGEFÜHRTE DOKUMENTE**

**In der Beschreibung aufgeführte Patentdokumente**

- DE 4024676 A1
- US 5586973 A
- US 4795424 A